(19) Europäisches Patentamt
European Patent Office
Office européen des brevets

(11) Veröffentlichungsnummer : **0 167 820**
**B1**

(12) # EUROPÄISCHE PATENTSCHRIFT

(45) Veröffentlichungstag der Patentschrift : **26.04.89**

(51) Int. Cl.⁴ : **C 07 D209/92**

(21) Anmeldenummer : **85106908.8**

(22) Anmeldetag : **04.06.85**

(54) **Verfahren zur Herstellung von gegebenenfalls substituierten Benz (c,d)-indol-2-onen(Naphtolactamen).**

(30) Priorität : **14.06.84 DE 3422076**
**01.12.84 DE 3443994**

(43) Veröffentlichungstag der Anmeldung :
**15.01.86 Patentblatt 86/03**

(45) Bekanntmachung des Hinweises auf die Patenterteilung : **26.04.89 Patentblatt 89/17**

(84) Benannte Vertragsstaaten :
**CH DE FR GB IT LI**

(56) Entgegenhaltungen :
**DE-A- 2 143 744**
**DE-B- 1 295 555**
**DE-C- 887 198**
**HOUBEN-WEYL,"Methoden der Organischen Chemie", Band E4, Seiten 741-784, 1983, GEORG THIEME VERLAG, Stuttgart, DE; K. FINDEISEN et al.:"Alkyl- und Aryl-isocyanate"**
**CHEMISCHE BERICHTE, Band 105, Nr. 6, 1972, Seiten 1926-1942, VERLAG CHEMIE, Weinheim, DE, 1942, F. EFFENBERGER et al.: "Die Umlagerung von Carbamidsäure-phenylestern-Synthesen von 2.4-Dioxo-3,4-dihydro-2H-1.3-benzoxazinen und Salicylamiden"**
**SYNTHESIS, Oktober 1982, Seiten 879-881, GEORG THIEME VERLAG, Stuttgart, DE; G. BALDUZZI et al. "Unusual Friedel-Crafts Reactions; 5. Synthesis of Salicylanilides via ortho-Aminocarbonylation of Phenols with Phenyl Isocyanate"**
**CHEMICAL ABSTRACTS, Band 67, Nr. 23, Seite 10217, Spalte 2, Abstract Nr. 108448z, Columbus, Ohio, US; & SU - A - 189 869 (DNEPROPETROVSK CHEMICOTECHNOLOGICAL INSTITUTE) 16.12.1966**

(73) Patentinhaber : **BAYER AG**
**Konzernverwaltung RP Patentabteilung**
**D-5090 Leverkusen 1 Bayerwerk (DE)**

(72) Erfinder : **Blank, Heinz Ulrich, Dr.**
**Am Geusfelde 35**
**D-5068 Odenthal (DE)**
Erfinder : **Silber, Gunter, Dr.**
**Gerstenkamp 21**
**D-5000 Köln 80 (DE)**
Erfinder : **Marzolph, Gerhard, Dr.**
**Semmelweisstrasse 87a**
**D-5000 Köln 80 (DE)**

**EP 0 167 820 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von N-substituierten Benz[c, d]-indol-2-onen (Naphtholactamen) durch Umsetzung von N-substituierten Carbamidsäurearylestern des N-substituierten α-Naphthylamins.

N-substituierte Naphtholactame, beispielsweise N-Alkyl- und N-Aryl-Derivate können erhalten werden, wenn man am Stickstoff nicht substituierte Naphtholactame mit Alkylierungs- bzw. Arylierungsreagenzien umsetzt. Beispielsweise sind in der SU-Patentschrift 242 908 (zitiert nach Chem. Abstr. 71, 81 022z (1969)) bzw, in der DE-OS 27 24 443 Dialkylsulfate in Gegenwart von Basen als Reagenzien beschrieben.

Weiterhin können aus 8-Halogen-1-naphthoesäuren durch Umsetzung mit primären Aminen N-alkylierte bzw. N-arylierte Naphtholactame erhalten werden (Zh. Org. Khim. 5, 478 (1969) und ibid. 7, 2186 (1971)).

N-Phenyl-benz[c, d]-indol-2-on wurde durch Umsetzung von N-Phenyl-N-α-naphthylamin mit Butyllithium in 8-Stellung des Naphthalinringsystems, nachfolgende Carboxylierung mit Kohlendioxid und spontanem Ringschluß erzeugt (Indian J. Chem. 7, 538 (1969)).

In EP 84 853 ist ferner ein Verfahren zur Herstellung von N-substituierten Benz[c,d]-indol-2-onen durch Umsetzung von 1,8-Naphtholactonen mit primären Aminen beschrieben. Dieses Verfahren erfordert in nachteiliger Weise einen hohen Überschuß an Amin und lange Reaktionszeiten bis zu 15 Stunden.

In GB 973 260 ist weiterhin ein Verfahren zur Herstellung von N-substituierten Benz[c,d]-indol-2-onen durch Cyclisierung von N-α-Naphthyl-N-alkyl-carbamoylchloriden in Gegenwart von $AlCl_3$ in indifferenten Lösungsmitteln beschrieben. In diesem Verfahren müssen die vom arbeitshygienischen Standpunkt her bedenklichen Carbamoylchloride eingesetzt werden, denen krebserregende Wirkung zugeschrieben wird.

Aus der SU-Patentschrift 189 869 (zitiert nach Chem. Abstr. 67, 108 448z (1967)) ist bekannt, daß sich Phenolate mit Phenylurethan zu Salicylsäureaniliden aminocarbonylieren lassen. Nach Zh. Org. Khim. 18, 1287 (1982) (zitiert nach Chem. Abstr. 97, 109 661 v (1982)) und Chem. Ber. 105, 1926 (1972) ist jedoch primär mit der Bildung eines Arylcarbamats zu rechnen, das in einem zweiten Reaktionsschritt im Sinne einer Fries-Umlagerung das Salicylsäureamid liefert. Das Vorhandensein einer phenolischen Hydroxylgruppe ist in solchen Reaktionen Vorbedingung.

DE-OS 21 43 744 beschreibt die Bildung von Dihydro-isochinolinonen durch Ringschluß von Carbaminsäureestern, deren N-Atom stets ein H-Atom trägt und über eine aliphatische Kette an einen Benzolkern gebunden ist. Da dieser Ringschluß auch durch die entsprechenden Isocyanate erfolgt, kann vermutet werden, daß aus den Carbamaten zunächst die Isocyanate gebildet werden, wozu jedoch ein N-ständiges H-Atom erforderlich ist.

Houben-Weyl, Methoden der Organischen Chemie, Band E 4 (1983), S.753/754 beschreibt die Bildung von Isocyanaten aus Carbamaten durch Abspaltung von Alkoholen, wozu ebenfalls ein N-ständiges H-Atom erforderlich ist.

DE-PS 887 198 beschreibt die Bildung von Naphthostyril und dessen am Kern durch Chlor, Methoxy bzw. Methyl substituierten Derivaten durch Cyclisierung von Naphthyl-1-isocyanat mit Friedel-Crafts-Katalysatoren, wobei nur NH-haltige Naphthostyril-Derivate erhältlich sind.

Schließlich ist aus Synthesis *1981*, 977 die Aminocarbonylierung von alkylsubstituierten Aromaten, wie Xylol oder Mesitylen, mit Ethylcarbamat in Gegenwart von $AlCl_3$ zu den entsprechenden Säureamiden bekannt. Urethane können jedoch nach Houben-Weyl, Bd. VIII, S. 167 (1952) unter Abspaltung von Alkohol in Isocyanate übergeführt werden, deren acylierende Wirkung unter Friedel-Crafts-Bedingungen beschrieben ist (Houben-Weyl, Bd. VIII, S. 135 (1952), so daß mit uneinheitlichem Reaktionsverlauf infolge einer solchen Abspaltung zu rechnen ist.

Es wurde nun ein Verfahren zur Herstellung von N-substituierten Benz [c, d]-indol-2-onen (Naphtholactamen) der Formel

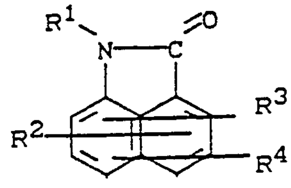

(VII)

worin

$R^1$ Phenyl, 2-, 3- oder 4-Pyridyl ; 2- oder 3-Pyrazinyl ; 2-, 4- oder 5-Pyrimidinyl ; 3- oder 4-Pyridazinyl ; 2-triazinyl ; 2-, 3-, 4-, 5-, 6-, 7- oder 8-Chinolinyl ; 1-, 3-, 4-, 5-, 6-, 7- oder 8-Isochinolinyl ; 2-, 3-, 5-, 6-, 7- oder 8-Chinoxalinyl ; 2-, 4-, 5-, 6-, 7- oder 8-Chinazolinyl ; 1,8-Naphthyridin-2-, -3-, -4-, -5-, -6- oder -7-yl ; 3-, 4-, 5-, 6-, 7- oder 8-Chinnolinyl ; 1-, 4-, 5-, 6-, 7- oder 8-Phthalazinyl ; 2-, 4-, 6- oder 7-Pteridinyl ; 1-, 2-, 3-, 4-, 6-, 7-, 8- oder 9-Phenazinyl ; 1-, 2-, 3-, 4-, 5-, 6-, 7-, 8- oder 9-Acridinyl ; 1-, 2-, 3-, 4-, 6-, 7-, 8-, 9- oder 10-Phenanthridinyl ; 2- oder 3-Pyrrolyl ; 1-, 2- oder 4-Imidazolyl ; 1-, 2-, 3-, 4-, 5-, 6- oder 7-Indolyl ; 3-, 4-, 5-, 6- oder 7-Indazolyl ; 2-, 6- oder 8-Purinyl. 2- oder 3-Thienyl ; 2- oder 3 Furyl ; 2-, 3-, 4-, 5-, 6 oder 7-Benzo[b]-thienyl ; 1-, 2-, 3-, 4-, 6-, 7-, 8- oder 9-Thianthrenyl ; 2-, 3-, 4-, 5-, 6- oder 7-[b]-furanyl ; 1-, 2-, 3-, 4-, 5-, 6-, 7-, 8- oder 9-Phenoxazinyl ; 1-, 2-, 3-, 4-, 6-, 7-, 8- oder 9-Phenothiazinyl oder $-CH_2-R^8$ darstellt, worin $R^8$ Wasserstoff, geradkettiges oder verzweigtes $C_1$-$C_3$-Alkyl oder Phenyl bedeutet und

$R^2$, $R^3$ und $R^4$ unabhängig voneinander für Wasserstoff, substituiertes oder nicht substituiertes geradkettiges oder verzweigtes $C_1$-$C_{20}$-Alkyl, $C_4$-$C_8$-Cycloalkyl, Phenyl, Naphthyl, Anthryl, Biphenyl, Benzyl, Phenyl-ethyl, Naphthyl-methyl, Naphthyl-ethyl, Anthryl-methyl, Anthryl-ethyl, Halogen, Cyano, Hydroxy, Formyl, $C_1$-$C_{10}$-Alkylcarbonylamino, Phenylamino, $C_1$-$C_{10}$-Alkoxycarbobyl, $C_1$-$C_{10}$-Alkylcarbonyloxy, Phenyloxycarbonyl, Phenylcarbonyloxy, $C_1$-$C_{10}$-Alkylcarbonyl, Phenylcarbonyl, $C_1$-$C_{10}$-Alkoxy, Phenyloxy, $C_1$-$C_{10}$-Alkylthio, Benzylthio, Phenylthio, $C_1$-$C_{10}$-Alkoxycarbonyloxy, $C_1$-$C_{10}$-Acylamino, Di-$C_1$-$C_{10}$-alkylamino, $C_1$-$C_{10}$-Alkylphenylamino, $C_1$-$C_{10}$-Alkylaminocarbonyloxy, $C_1$-$C_{10}$-Alkylsulfonylamino, Ureido, N-$C_1$-$C_{10}$-Alkyl-Ureido, Phenyloxycarbonylamion, $C_1$-$C_{10}$-Alkoxycarbonylamino, Carbamoyl, N-$C_1$-$C_{10}$-Alkyl-carbamoyl, Di-N,N-$C_1$-$C_{10}$-alkylcarbamoyl, N-$C_1$-$C_{10}$-Alkyl-N-phenyl-carbamoyl, Sulfamoyl, N-$C_1$-$C_{10}$-Alkyl-sulfamoyl, Di-N,N-$C_1$-$C_{10}$-alkyl-sulfamoyl, Phenyloxysulfonyl, $C_1$-$C_{10}$-Alkoxysulfonyl, Nitro, $C_1$-$C_{10}$-Alkylsulfonyl, Benzylsulfonyl oder Phenylsulfonyl stehen, wobei weiterhin zwei der Reste $R^2$, $R^3$ und $R^4$ gemeinsam einen anellierten aromatichen oder cycloaliphatischen Ring darstellen können,

gefunden, das dadurch gekennzeichnet ist, daß man N-subtituierte N-α-Naphthyl-carbamidsäureester der Formel

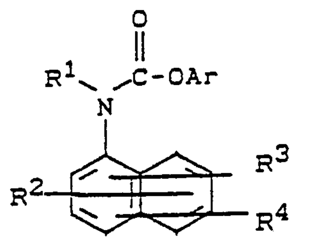

(I)

in der

$R^1$ bis $R^4$ die genannte Bedeutung haben und

Ar Phenyl, das einfach oder mehrfach durch $C_1$-$C_4$-Alkyl, $C_1$-$C_4$-Alkoxy, Fluor, Chlor, Brom oder Cyano substituiert sein kann, oder Naphthyl bedeutet,

in Gegenwart von 1-5 Mol eines Friedel-Crafts-Katalysators pro Mol des Carbamidsäureesters in einem für Friedel-Crafts-Reaktionen geeigneten Lösungsmittel bei erhöhter Temperatur umsetzt.

Als geradkettiges oder verzweigtes Alkyl sei beispielsweise solches mit 1-20, bevorzugt 1-10, besonders bevorzugt 1-4 Kohlenstoffatomen genannt, wie Methyl, Ethyl, geradkettiges oder verzweigtes Propyl, Butyl, Amyl, Hexyl, Octyl, Decyl, Dodecyl, Hexadecyl oder Eikosyl.

Als Cycloalkyl sei beispielsweise solches mit 4-8, bevorzugt 5-6 im Ring angeordneten Kohlenstoffatomen genannt, das gegebenenfalls eine oder zwei Methyl- oder Ethylgruppen tragen kann, wie Cyclobutyl, Cyclopentyl, Cyclohexyl, Cycloheptyl, Cyclooctyl, Methylcyclopentyl, Ethyl-cyclopentyl, Methyl-cyclohexyl- oder Ethyl-cyclohexyl.

Die genannten Alkyl- bzw. Cycloalkyl-Reste können substituiert sein, beispielsweise durch Halogen, Hydroxy, Cyano, $C_1$-$C_{10}$-Alkoxycarbonyl, Phenyloxycarbonyl, $C_1$-$C_{10}$-Alkoxy, Phenyloxy, $C_1$-$C_{10}$-Alkylcarbonyloxy oder Phenylcarbonyloxy. In bevorzugter Weise tragen die genannten Alkyl- oder Cycloalkylgruppen keine solche Substituenten.

Von den für $R^1$ genannten heteroaromatischen Resten kommen in bevorzugter Weise Pyridyl, Pyrimidinyl, Triazinyl, Chinolinyl, Acridinyl, Thienyl, Thiazolyl, Phenoxazinyl oder Phenothiazinyl mit den obengenannten Positionsnummern der Ringatome in Frage. In besonders bevorzugter Weise sei Pyridyl, Pyrimidinyl, Triazinyl oder Thiazolyl genannt.

Die genannten Aryl- und Aralkyl-Reste sowie die heterocyclischen Reste können durch Halogen, Cyano, $C_1$-$C_{10}$-Alkoxycarbonyl, Phenyloxycarbonyl, $C_1$-$C_{10}$-Alkylcarbonyloxy, Phenylcarbonyloxy, $C_1$-$C_{10}$-Alkylcarbonyl, Phenylcarbonyl, $C_1$-$C_{10}$-Alkoxy, Phenyloxy, $C_1$-$C_{10}$-Acylamino, $C_1$-$C_{10}$-Dialkylamino, $C_1$-$C_{10}$-Alkylarylamino, $C_1$-$C_{10}$-Alkylsulfonyl oder Phenylsulfonyl sowie $C_1$-$C_4$-Alkyl substituiert sein ; bevorzugt ist eine Substitution mit Fluor, Chlor, Brom, Cyano, Methoxy, Ethoxy, Ethyl oder Methyl. Solche Substituenten können auch mehrfach auf den genannten Aryl- oder Aralkyl-Resten sowie den heterocyclischen Resten angeordnet sein.

Für den Fall, daß $R^1$ Phenyl bedeutet kann dieses außer den genannten Substituenten auch durch die Gruppe A substituiert sein, die auch zu den bevorzugten Substituenten gehört. Die Gruppe A hat die folgende Struktur

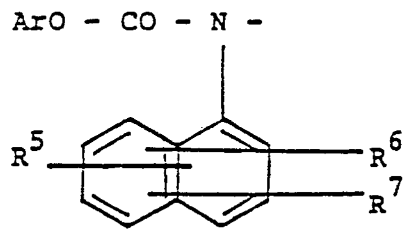

(A)

worin

Ar den obengenannten Bedeutungsumfang hat und $R^5$, $R^6$ und $R^7$ den Bedeutungsumfang von $R^2$, $R^3$ und $R^4$, jedoch unabhängig untereinander und unabhängig von $R^2$, $R^3$ und $R^4$, haben.

Eingeschränkte Bedeutungsumfänge von $R^5$, $R^6$ und $R^7$ folgen jeweils dem Umfang von $R^2$, $R^3$ und $R^4$. Die Gruppe A steht in o-, m- oder p-Stellung zum Verknüpfungsort von Aryl mit dem N-Atom, bevorzugt in m- oder p-Stellung, besonders bevorzugt in p-Stellung.

Als Halogen sei beispielsweise Fluor, Chlor, Brom, Iod, bevorzugt Fluor, Chlor, Brom, besonders bevorzugt Chlor oder Brom, genannt.

Die in den Alkoxycarbonyl-, Alkylcarbonyloxy-, Alkylcarbonyl-, Alkoxy-, Acylamino-, Dialkylamino-, Alkylarylamino- und Alkylsulfonyl-Gruppen genannten Alkylreste haben beispielsweise 1-10, bevorzugt 1-4 C-Atome und stellen besonders bevorzugt Methyl oder Ethyl dar und können, wie oben dargestellt, substituiert sein. In den zu den genannten Gruppen analogen Aryl-Gruppen stellt Aryl Phenyl oder Naphthyl, bevorzugt Phenyl dar. Solche Aryl-Gruppen können in der oben angegebenen Weise substituiert sein.

Bevorzugte Substrate der Formel (I) sind solche, in denen anstelle der Reste $R^2$, $R^3$ und $R^4$ die Reste $R^{12}$, $R^{13}$ und $R^{14}$ treten, wobei die zuletztgenannten Reste unabhängig voneinander für Wasserstoff, Fluor, Chlor, Brom, Cyano, Aryloxycarbonylamino, $C_1$-$C_4$-Alkyl oder $C_1$-$C_4$-Alkoxy stehen.

Unter den bevorzugten Substraten der Formel (I), wie sie soeben beschrieben wurden, sind besonders bevorzugt solche, in denen sowohl $R^2$, $R^3$, $R^4$ als auch Ar die genannte eingeschränkte Bedeutung besitzen. Solche besonders bevorzugten Substrate sind beispielsweise solche der Formel

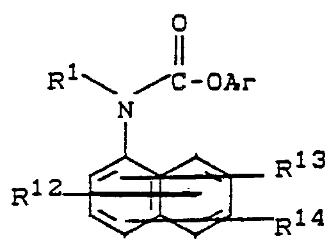

(II)

in der $R^1$, $R^{12}$, $R^{13}$, $R^{14}$ und $Ar^1$ unabhängig voneinander die genannte Bedeutung besitzen.

In ganz besonders bevorzugter Form seien für das erfindungsgemäße Verfahren Substrate der Formel

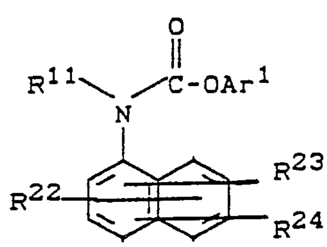

(III)

genannt, in der

$R^{11}$ für Phenyl, Pyridyl, Pyrimidinyl, Triazinyl, Chinolinyl, Acridinyl, Thienyl, Thiazolyl, Phenoxazinyl, Phenothiazinyl oder $C_1$-$C_4$-Alkyl steht,

$R^{22}$, $R^{23}$ und $R^{24}$ unabhängig voneinander Wasserstoff, Chlor, Brom, Methyl oder Ethyl bedeuten und

$Ar^1$ für gegebenenfalls durch Chlor, Brom, Methyl oder Ethyl substituiertes Phenyl steht.

Das erfindungsgemäße Verfahren wird in Gegenwart eines dem Fachmann bekannten Friedel-Crafts-Katalysators durchgeführt. Solche Katalysatoren sind beispielsweise Aluminiumchlorid, Aluminiumbromid, Eisen-(III)-chlorid, Eisen-(III)-bromid, Bortrifluorid, Zinntetrachlorid, Zinkchlorid, Zinkbromid, Antimonchloride oder Antimonbromide. Das erfindungsgemäße Verfahren wird bevorzugt in Gegenwart von Aluminiumchlorid oder Aluminiumbromid, besonders bevorzugt in Gegenwart von Aluminiumchlorid,

durchgeführt. Der Friedel-Crafts-Katalysator wird hierbei in einer Menge von 1-5 Mol, bevorzugt 1,5-3,5 Mol, besonders bevorzugt 1,8-2,5 Mol pro Mol des Carbamidsäureesters eingesetzt. Der Einsatz von mehr als 5 Mol Friedel-Crafts-Katalysator ist grundsätzlich möglich, bringt jedoch keine weiteren Vorteile. In Fällen mit 2 Carbamidsäureester-Gruppen pro Molekül des Ausgangsstoffes sind die genannten Mengen des Friedel-Crafts-Katalysators zu verdoppeln. Der Friedel-Crafts-Katalysator wird im erfindungsgemäßen Verfahren in gelöster oder mindestens zum Teil gelöster Form eingesetzt, wobei im letzteren Fall der Rest des Katalysators als Suspension vorliegt. In bevorzugter Weise wird in Gegenwart des gelösten Katalysators gearbeitet.

Als Lösungsmittel für das erfindungsgemäße Verfahren kommen alle die in Frage, die dem Fachmann als für Friedel-Crafts-Reaktionen geeignet bekannt sind. Solche Lösungsmittel sind beispielsweise halogenierte aromatische oder aliphatische Kohlenwasserstoffe, wie Chlorbenzol, Brombenzol, Chlortoluole, Dichlorbenzole, Dibrombenzole, Dichlortoluole, Trichlorbenzole, Methylenchlorid, Dichlorethan oder Tetrachlorethan. In bevorzugter Weise werden die genannten halogenierten Aromaten eingesetzt, in besonders bevorzugter Weise sei 1,2,4-Trichlorbenzol, Dichlortoluole (Isomerengemisch) und o-Dichlorbenzol genannt. Die Menge des Lösungsmittels ist für die Durchführung des erfindungsgemäßen Verfahrens nicht kritisch und daher in einem weiten Bereich variierbar. Man arbeitet jedoch zweckmäßigerweise in einer Mindestmenge, die ausreicht, um bei der gewählten Temperatur ein homogenes Reaktionsgemisch zu gewährleisten. Zur Erzielung einer möglichst hohen Raum-Zeit-Ausbeute arbeitet man in möglichst konzentrierten Lösungen. Am Beispiel der Verwendung von o-Dichlorbenzol als Lösungsmittel sei für die Gesamtmenge an Lösungsmittel das 2-8-fache, bevorzugt 2,5-6-fache der Katalysatormenge genannt. Von dieser Gesamtmenge an Lösungsmittel wird ein Teil für das Auflösen des Katalysators und gegebenenfalls ein weiterer Teil für das Auflösen des Carbamidsäureesters eingesetzt.

Das erfindungsgemäße Verfahren wird bei erhöhter Temperatur durchgeführt. Als solche sei beispielsweise der Bereich von 100-250 °C, bevorzugt 150-220 °C, besonders bevorzugt 160-190 °C genannt.

Die Reaktionspartner werden im allgemeinen in gelöster oder suspendierter Form im Rahmen des angegebenen Temperaturbereiches so zusammengegeben, daß die gewünschte Temperatur eingehalten werden kann. Nach Beendigung der Reaktion, die beispielsweise in einer dem Fachmann geläufigen Weise durch chromatographische Untersuchung von entnommenen Proben des Reaktionsgemisches verfolgt werden kann, kann vor der Aufarbeitung noch eine geringe Nachreaktionszeit angesetzt werden.

Die Reaktion des erfindungsgemäßen Verfahrens ist nicht abhängig von der Einhaltung eines bestimmten äußeren Druckes, so daß bei Normaldruck, Überdruck, aber auch Unterdruck gearbeitet werden kann. In bevorzugter Weise wird immer dann, wenn es die Siedepunkte der Reaktions-partner erlauben, bei Normaldruck gearbeitet. Bei der Anwendung höherer Reaktionstemperaturen im angegebenen Temperaturbereich und dem gleichzeitigen Einsatz niedrig siedender Lösungs- bzw. Suspendiermittel wird die Reaktion in einer dem Fachmann geläufigen Weise bei erhöhtem Druck durchgeführt. Als erhöhter Druck kommt im allgemeinen der Eigendruck des Reaktionsgemisches bei der Reaktionstemperatur in Betracht. Ein zusätzlicher Druck durch Zugabe eines Inertgases, wie Stickstoff, Luft oder Wasserstoff ist möglich, jedoch nicht erforderlich.

Die Reihenfolge des Zusammengebens von Carbamidsäureester und Friedel-Crafts-Katalysator ist grundsätzlich beliebig ; in bevorzugter Weise wird jedoch der Katalysator in einem Teil des Lösungsmittels vorgelegt und der Carbamidsäureester in gelöster Form (im restlichen Teil des Lösungsmittels) oder als Schmelze zugesetzt. Als Teil des Lösungsmittels für das Vorlegen des Katalysators sei beispielsweise die zwei bis fünffache, bevorzugt 2,5-bis 3-fache Menge an Lösungsmittel, bezogen auf die Menge des Friedel-Crafts-Katalysators, genannt. Hierbei werden in einer bevorzugten Weise der Katalysator und das Lösungsmittel solange auf die gewünschte Reaktionstemperatur erhitzt, bis eine homogene Lösung vorliegt. Anschließend wird der Carbamidsäureester so zudosiert, daß die gewünschte Reaktionstemperatur eingehalten werden kann. Wird der Carbamidsäureester in der bevorzugten Weise als Schmelze zudosiert, kann in vorteilhafter Weise die Gesamtmenge des Lösungsmittels auf die zur Auflösung des Katalysators erforderliche Menge begrenzt werden, wodurch die Raum-Zeit-Ausbeute in vorteilhafter Weise verbessert wird. Für das Suspendieren oder Auflösen des Katalysators und für das Zudosieren des Carbamidsäureesters in gelöster Form wird in bevorzugter Weise das gleiche Lösungsmittel verwendet.

Zur Aufarbeitung des Reaktionsansatzes wird der Friedel-Crafts-Katalysator durch Zusatz von Wasser oder verdünnter Salzsäure bei 0 bis 150 °C hydrolysiert. Das Gemisch der wäßrigen und organischen Phase kann anschließend einer Klärfiltration, beispielsweise über übliche säurebeständige Filter, zugeführt werden, gegebenenfalls nach Zusatz von handelsüblichen Klär- und/oder Filtrierhilfsmitteln, wie Cellulosepulver, Tonsil, Aktivkohle und/oder Kieselgur. Die Phasentrennung wird vorteilhaft bei 50-100 °C, bevorzugt 60-100 °C durchgeführt. Die organische Phase kann in üblicher Weise mit Wasser oder 1-10-gew.- %iger Natriumhydrogencarbonatlösung gewaschen werden. Das aus der organischen Phase beispielsweise destillativ zurückgewonnene Lösungsmittel kann nach Trocknung erneut im erfindungsgemäßen Verfahren verwendet werden. Aus dem Destillationsrückstand der organischen Phase kann das gewünschte gegebenenfalls substituierte Naphtholactam durch Kristallisation oder Vakuumdestillation in reiner Form gewonnen werden.

In einer weiteren Ausgestaltung der Aufarbeitung des Reaktionsansatzes wird bei Verwendung von Aluminium halogenid als Friedel-Crafts-Katalysator dem Reaktionsansatz bei 80-120 °C 1,0-1,3 Mol

Alkalihalogenid, beispielweise Kochsalz, pro Mol Aluminiumhalogenid zugesetzt. Anschließend kann der größte Teil des verwendeten Lösungsmittels (bis zu etwa 80 % der Gesamtmenge) direkt abdestilliert und ohne weitere Reinigung wieder für die Reaktion eingesetzt werden. Der nach dieser Destillation aufkonzentrierte Reaktionsansatz wird anschließend mit Wasser oder verdünnter Salzsäure hydrolysiert und in der oben beschriebenen Weise weiter aufgearbeitet.

Die im erfindungsgemäßen Verfahren eingesetzten Carbamidsäureester können nach bekannten Methoden hergestellt werden (Ullmanns Encyclopädie der technischen Chemie, 4. Auflage, Band 9, Seite 118 ff ; Houben-Weyl, Methoden der organischen Chemie, 4. Auflage, Band VIII, Seite 137 ff.). So kann beispielsweise ein Chlorameisensäurearylester der Formel (IV), in der Ar die obengenannte Bedeutung hat, mit einem α-Naphtylamin der Formel (V), in dem $R^1$, $R^2$, $R^3$ und $R^4$ die obengenannte Bedeutung haben, gegebenenfalls in einem geeigneten Lösungsmittel und in Gegenwart einer Base umgesetzt werden :

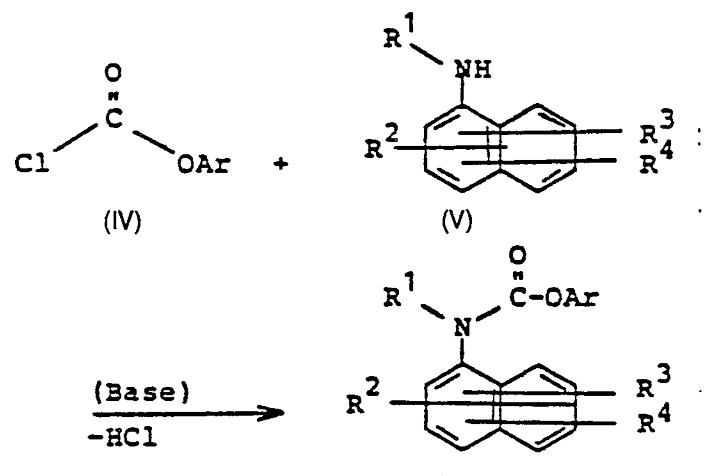


(IV) (V)

(I)

Geeignete Basen sind anorganische Basen, wie Natronlauge, Kalilauge, Kalkmilch, Alkalicarbonate, Alkalihydrogencarbonate, Calciumcarbonat, aufgeschlemmtes Magnesiumoxid und ähnliche, sowie organische tertiäre Amine, wie Triethylamin. Als Lösungsmittel kommen aliphatische oder aromatische Kohlenwasserstoffe und deren Halogensubstitutionsprodukte in Frage, wie Benzol, Toluol, Ethylbenzol, Xylol, Petroleumfraktionen, Kerosin, Cyclohexan, Chlorbenzol, o-Dichlorbenzol, Trichlorbenzol, Chlortoluole oder Dichlortoluole, gegebenenfalls als Isomerengemisch, sowie Methylenchlorid, Dichlorethan oder andere. In vorteilhafter Weise verwendet man zur Herstellung der Carbamidsäureester das gleiche Lösungsmittel, das anschließend für das erfindungsgemäße Verfahren einsetzbar ist. In diesem Fall kann die Lösung des Carbamidsäureesters nach Abfiltrieren des aus der Base (Hilfsbase) und dem abgespaltenen Chlorwasserstoff gebildeten Chlorids oder Hydrochlorids direkt und ohne Zwischenisolierung des Carbamidsäureesters in das erfindungsgemäße Verfahren eingesetzt werden.

In einer weiteren Ausgestaltung des erfindungsgemäßen Verfahrens wird die Umsetzung des Chlorameisensäure-arylesters der Formel (IV) mit dem gegebenenfalls substituierten α-Naphthylamin der Formel (V) zum erfindungsgemäß einzusetzenden, gegebenenfalls substituierten Carbamidsäureester der Formel (I) bei erhöhter Temperatur, beispielsweise 50-180 °C, bevorzugt 80-150 °C, gegebenenfalls in einem der obengenannten Lösungsmittel und, sofern erforderlich, unter Druck, durchgeführt, wobei auf jede Hilfsbase verzichtet werden kann. Hierbei wird ein Molverhältnis von α-Naphthylamin der Formel (V) zum Chlorameisensäurephenylester der Formel (IV) von 1 :1,0-1,2, bevorzugt 1 :1,05-1,1 angewendet, so daß das α-Naphthylamin der Formel (V) nicht im Überschuß, sondern bevorzugt im geringen Unterschuß eingesetzt wird. Das unter diesen Reaktionsbedingungen zunächst entstehende Hydrochlorid des α-Naphthylamins (V) wird unter Freisetzen von Chlorwasserstoff wieder in das freie α-Naphthylamin zurückgespalten, welches sodann weiter umgesetzt werden kann. Die Lösung des Carbamidsäureesters kann, gegebenenfalls nach Aufkonzentrieren, ohne zusätzliche Reinigung in das erfindungsgemäße Verfahren eingesetzt werden.

Die Erfindung betrifft daher weiterhin ein Verfahren zur Herstellung von gegebenenfalls substituierten Benz [c, d]-indol-2-onen (Naphtholactamen), das dadurch gekennzeichnet ist, daß man in einer ersten Stufe ein gegebenenfalls substituiertes α-Naphthylamin mit 1,0-1,2 Mol Chlorameisensäurearylester pro Mol α-Napthylamin bei erhöhter Temperatur in einem für Friedel-Crafts-Reaktionen geeigneten Lösungsmittel ohne Hilfsbase umsetzt und diese Lösung, gegebenenfalls nach Aufkonzentrieren, in einer zweiten Stufe zu einer Lösung von Aluminiumhalogenid im gleichen Lösungsmittel bei erhöhter Temperatur zusetzt und dann zum gegebenenfalls substituierten Benz[c, d]-indol-2-on (Naphtholactam) in der oben genannten Weise umsetzt.

Als gegebenenfalls substituierte α-Naphthylamine kommen beispielsweise solche der Formel (V) und als Chlorameisensäurearylester beispielsweise solche der Formel (IV) in Frage, worin $R^1$, $R^2$, $R^3$ $R^4$ und Ar die oben angegebene Bedeutung haben.

Das im erfindungsgemäßen Verfahren abgespaltene Phenol der Formel

ArOH (VI),

worin Ar die obengenannte Bedeutung hat, erscheint je nach seinen Löslichkeitsverhältnissen bei der Aufarbeitung zum Teil in der wäßrigen und zum Teil in der organischen Phase. Es kann in einer dem Fachmann üblichen Weise durch Extraktion und/oder Destillation aus der wäßrigen Phase gewonnen werden. Die in der organischen Phase verbliebenen Mengen dieses Phenols werden bei der destillativen Aufarbeitung als eigene Fraktion gewonnen. Die zurückgewonnenen Mengen des Phenols können, gegebenenfalls nach gesonderter Reinigung, erneut zur Herstellung von Chlorameisensäurearylester der Formel (IV) eingesetzt werden.

Das erfindungsgemäße Verfahren erlaubt die vorteilhafte Herstellung von N-substituierten Benz[c, d]-indol-2-onen (Naphtholactamen) der Formel

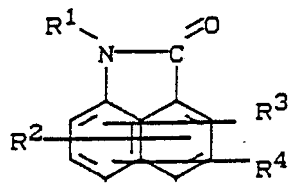

(VII)

in der $R^1$, $R^2$, $R^3$ und $R^4$ die obenangegebene Bedeutung haben.

Das erfindungsgemäße Verfahren erlaubt demnach die vorteilhafte Herstellung von Verbindungen der Formel (I), in denen der Rest $R^1$ verschieden von Wasserstoff ist. Das erfindungsgemäße Verfahren liefert diese Verbindungen auf einfachere und wirtschaftlichere Weise unter Vermeidung arbeitshygienisch bedenklicher Zwischenprodukte in hoher Reinheit und in guten Ausbeuten.

Das erfindungsgemäße Verfahren ist überraschend und war in keiner Weise vorhersehbar. Im Sinne der eingangs genannten Literatur war davon auszugehen, daß Carbamidsäurearylester im Sinne einer Fries-Verschiebung zu Salicylsäureamid umlagern. In Übereinstimmung hiermit findet sich in der Literatur kein Hinweis auf die Carboxamidierung von Aromaten mit Carbamaten, insbesondere nicht mit N,N-disubstituierten Carbamaten.

Es mußte also mit einer unübersichtlichen Reaktion und dem Entstehen unerwünschter Nebenprodukte gerechnet werden. Besonders überraschend war daher, daß Carbamidsäureester der aromatischen Reihe im erfindungsgemäßen Verfahren einen einheitlichen Verlauf gewährleisten. Entsprechende Carbamidsäurealkylester spalten nämlich unter den Bedingungen des erfindungsgemäßen Verfahrens in die α-Naphtylamin-Komponente zurück.

Die erfindungsgemäß herstellbaren Benz[c, d]-indol-2-one (Naphtholactame), insbesondere die N-Phenyl- und die N-Alkyl-substituierten, sind bekannte Zwischenprodukte zur Herstellung wertvoller Farbstoffe (GB-973 259, DE-AS-1 170 569, BE-671 139 und BE-695 292).

## Beispiel 1

166,8 g (1,25 Mol) Aluminiumchlorid und 474 g o-Dichlorbenzol wurden in einer Rührapparatur, versehen mit Tropftrichter, Rückflußkühler mit Gasableitungsrohr und Thermometer, vorgelegt. Man erhitzte unter Rühren auf 160 °C, wobei Aluminiumchlorid in Lösung ging. Als eine homogene Lösung vorlag, tropfte man eine Lösung von 145,68 g (0,5 Mol) N-Ethyl-N-α-naphthylcarbamidsäure-phenylester in 340 g o-Dichlorbenzol innerhalb von 60 Minuten zu. Nach beendeter Zugabe des Carbamats wurde noch 4 h bei 160 °C gerührt. Man kühlte auf 40 °C ab und trug den Reaktionsansatz auf eine Mischung von 0,5 Ltr Wasser und 1,5 kg Eis aus. Man rührte 1 h bei 60 °C und trennte die Phasen bei dieser Temperatur. Die organische Phase wurde anschließend einmal mit 1,5 Ltr Wasser, dann mit 1,5 Ltr 3 %iger Natriumcarbonatlösung gewaschen, wobei man die Phasen jeweils bei 60 °C trennte. Man erhielt 975,0 g organische Phase. Nach Hochdruck-Flüssigkeits-Chromatographie (HFC)-Analyse enthielt die Lösung 8,37 % ≙ 81,6 g N-Ethyl-benz [c, d]-indol-2-on.

Die vereinigten wäßrigen Phasen wurden zweimal mit je 450 g o-Dichlorbenzol extrahiert. Nach Wäsche der organischen Phase mit 3 %iger Natriumcarbonatlösung ergaben sich 898 g organische Lösung mit einem Gehalt von 0,48 % ≙ 4,3 g N-Ethyl-benz [c, d]-indol-2-on lt HFC.

Die Gesamtausbeute betrug damit 85,9 g N-Ethyl-benz [c, d]-indol-2-on ≙ 87 % der theoretischen Ausbeute.

Die vereinigten organischen Phasen wurden nach Trocknen über Natriumsulfat und Entfernen des Lösungsmittels am Rotationsverdampfer einer Vakuumdestillation unterworfen. Nach einem Vorlauf von 14,4 g gingen bei einer Temperatur von 146-154 °C/1,3-1,4 mbar 78,7 g gelbes Produkt über. Das Material erstarrte kristallin im Vorlagekolben und schmolz dann bei 62-65 °C. Laut HFC betrug die Reinheit 92,2 %. Die isolierte Ausbeute belief sich demnach auf 74 % der theoretischen Ausbeute.

Beispiel 2 (Vergleichsbeispiel)

Gearbeitet wurde analog Beispiel 1, jedoch unter Verwendung von 27,51 g (0,12 Mol) N-Ethyl-N-α-naphthyl-carbamidsäure-methylester in 64,2 g o-Dichlorbenzol und 41,1 g (0,31 Mol) Aluminiumchlorid in 117 g o-Dichlorbenzol. Eine gaschromatographische (GC) Analyse des abreagierten rohen Ansatzes zeigte 52,3 % N-Ethyl-N-α-naphthylamin sowie zahlreiche Nebenkomponenten. Das gewünschte N-Ethyl-benz [c, d]-indol-2-on konnte nur in Spuren nachgewiesen werden (< 1 %).

Beispiel 3 (Vergleichsbeispiel)

Gearbeitet wurde analog Beispiel 2, jedoch unter Verwendung von 30 g (0,12 Mol) N-Ethyl-N-α-naphthyl-carbamidsäure-ethylester in 70 g o-Dichlorbenzol. GC-Analyse des ausreagierten rohen Ansatzes : 50,8 % N-Ethyl-N-α-naphtylamin sowie zahlreiche Unbekannte. Das gewünschte N-Ethyl-benz [c, d]-indol-2-on konnte nicht nachgewiesen werden.

Beispiele 4 bis 7

In einer Reaktionsapparatur wie in Beispiel 1 wurden 41,1 g (0,31 Mol) Aluminiumchlorid und 117 g o-Dichlorbenzol vorgelegt. Man erhitzte auf die jeweilige Reaktionstemperatur (vgl. Tabelle 1) und dosierte innerhalb einer Stunde 34,96 g (0,12 Mol) N-Ethyl-N-α-naphthylcarbamidsäure-phenylester, gelöst in 82 g o-Dichlorbenzol, zu. Dann wurde bei Reaktionstemperatur 6 h gerührt. Die Ausbeuten wurden aus dem Gehalt der Rohlösung (bestimmt durch HFC) ermittelt.

Tabelle 1

| Beispiel | Temperatur (°C) | Gewicht der Reaktions-lösung (g) | Gehalt an Produkt lt. HFC (Gew.-%) | Ausbeute (% d.theor.Ausbeute) |
|---|---|---|---|---|
| 4 | 140 | 273,0 | 5,72 | 66 |
| 5 | 180[1)] | 265,8 | 7,80 | 88 |
| 6 | 200[2)] | 473,5 | 7,94 | 79 |
| 7 | 220[2)] | 465,9 | 7,65 | 75 |

1) Reaktionszeit nach Dosierende 4 h

2) Durchführung der Reaktion in einem Autoklaven. Doppelter Ansatz, Zupumpen der Carbamatlösung, Nachreaktion nach Dosierende 4 h.

Beispiel 8

In einer Reaktionsapparatur, wie unter Beispiel 1 beschrieben, wurden 32,0 g (0,24 Mol) Aluminiumchlorid und 91 g o-Dichlorbenzol vorgelegt, die Mischung unter Rühren auf 160 °C erhitzt und bei dieser Temperatur eine Lösung von 34,96 g (0,12 Mol) N-Ethyl-N-α-naphthylcarbamidsäurephenylester in 82 g o-Dichlorbenzol zugetropft. Anschließend ließ man bei Reaktionstemperatur 6 h nachrühren.

Nach Abkühlen erhielt man 234,4 g Rohlösung mit einem Gehalt von 8,53 % ≙ 19,99 g N-Ethyl-benz[c, d]-indol-2-on. Ausbeute : 84 % der theoretischen Ausbeute.

Beispiele 9 bis 11

Gearbeitet wurde wie in Beispiel 8, jedoch mit geändertem Molverhältnis des Carbamidsäureesters zu Aluminiumchlorid (vgl. Tabelle 2). Die Lösungsmittelmenge für Aluminiumchlorid wurde so berechnet, daß vor dem Zudosieren der Phenylcarbamatlösung eine 26 %ige Lösung von Aluminiumchlorid in o-Dichlorbenzol vorlag.

(Siehe Tabelle 2 Seite 10 f.)

Tabelle 2

| Beispiel | Molverhältnis Carbamat:$AlCl_3$ | Lösungsmittelmenge für $AlCl_3$ (g) | Gewicht der Reaktionslösung (g) | Gehalt an Produkt (Gew.-%) | Ausbeute (% d. theor. Ausbeute) |
|---|---|---|---|---|---|
| 9 | 1:1,9 | 86,5 | 229,7 | 8,24 | 80 |
| 10 | 1:1,8 | 82,0 | 224,0 | 7,39 | 70 |
| 11 | 1:1,7 | 77,4 | 216,9 | 6,83 | 63 |

Beispiel 12

Gearbeitet wurde wie in Beispiel 8, jedoch wurden zum Lösen von $AlCl_3$ 74,7 g, zum Lösen des Phenylcarbamats 59,5 g o-Dichlorbenzol eingesetzt.
Gesamtgewicht des ausreagierten Ansatzes : 196,6 g
Gehalt an N-Ethyl-benz[c, d]-indol-2-on : 10,09 %
Ausbeute : 84 % der theoretischen Ausbeute

Beispiel 13

Gearbeitet wurde wie in Beispiel 12, jedoch wurden zum Suspendieren von Aluminiumchlorid lediglich 48,0 g o-Dichlorbenzol eingesetzt.
Gewicht des ausreagierten Ansatzes : 170,3 g
Gehalt an N-Ethyl-benz[c, d]-indol-2-on : 11,12 %
Ausbeute : 80 % der theoretischen Ausbeute

Beispiel 14

Gearbeitet wurde wie in Beispiel 12, jedoch wurde das Phenylcarbamat ohne Lösungsmittel in geschmolzener Form zudosiert.
Gewicht des ausreagierten Ansatzes : 138,1 g
Gehalt an N-Ethyl-benz[c, d]-indol-2-on : 14,74 %
Ausbeute : 86 % der theoretischen Ausbeute

Beispiel 15

a) 171,24 g (0,965 Mol) N-Ethyl-N-α-naphthylamin 96,5 %ig wurden in 780 g o-Dichlorbenzol vorgelegt und die Lösung unter Rühren auf 100 °C erhitzt. Bei dieser Temperatur ließ man innerhalb 1 Stunde 164,4 g (1,05 Mol) Chlorameisensäure-phenylester zutropfen. Die anfänglich klare Lösung trübte sich allmählich unter Ausscheidung eines farblosen Niederschlags ; zusätzlich setzte eine lebhafte Gasentwicklung ein. Nach beendeter Zugabe wurde solange bei 105 bis 110 °C gerührt, bis wieder eine klare Lösung vorlag und die Gasentwicklung beendet war. Dies dauerte ca. 5 h. Nach dünnschichtchromatographischer Analyse (DC) hatte ein quantitativer Umsatz stattgefunden. Man destillierte daraufhin bei ca. 110 °C/100 mbar 100g o-Dichlorbenzol ab.
Der Rückstand (979,1 g) enthielt lt. HFC 281 g Phenyl-N-ethyl-N-α-naphthylcarbamat.
b) 487,8 g der oben hergestellten Lösung wurden bei 180 °C innerhalb von 1 h zu einer gut gerührten Lösung von 133,34 g (1,0 Mol) $AlCl_3$ in 380 g o-Dichlorbenzol zudosiert. Anschließend wurde 2 Stunden bei 180 °C nachgerührt, auf 100 °C abgekühlt und unter kräftigem Rühren 61,4 g (1,05 Mol) Natriumchlorid zugesetzt. Dann wurde auf 120 °C erhitzt und bei ca. 150 mbar insgesamt 545 g o-Dichlorbenzol abdestilliert. Nach Abkühlen trug man den Destillationssumpf auf 400 g Eis aus, erwärmte das 2-Phasen-Gemisch auf 60 °C und trennte bei dieser Temperatur die Phasen. Die organische Phase wurde einmal mit 300 ml Wasser und einmal mit 300 ml 3 %iger Sodalösung gewaschen, wobei man die Phasen jeweils bei 60 °C trennte. Man erhielt 254,8 g organische Lösung. Nach HFC enthielt die Lösung 32,72 Gew.- % N-Ethyl-benz[c, d]-indol-2-on ≙ 83,37 g ≙ 88 % der theoretischen Ausbeute, bezogen auf eingesetztes Carbamat.

Beispiel 16

a) N-α-Naphthyl-N-phenyl-carbamidsäure-phenylester

131,56 g (0,6 Mol) N-α-Naphthyl-N-phenylamin wurden in 470 g o-Dichlorbenzol gelöst und die Lösung auf 100 °C erhitzt ; unter Rühren wurden 98,28 g (0,63 Mol) Chlorameisensäure-phenylester in 1 Stunde zugetropft. Es fiel in geringer Menge ein Niederschlag aus. Nach beendetem Zutropfen wurde auf 120-130 °C erhitzt, wobei eine lebhafte Gasentwicklung auftrat. Man ließ ca. 4 Stunden bei dieser Temperatur rühren, danach war die Gasentwicklung beendet, und es lag wieder eine klare, dunkelgefärbte Lösung vor. Anschließend wurde das Lösungsmittel im Vakuum abdestilliert und die zurückbleibende Schmelze in 200 ml Toluol gelöst. Man kühlte auf 10 °C ab, setzte n-Hexan bis zur beginnenden Kristallisation zu und ließ im Eis-Kochsalz-Bad kristallisieren.
Nach Absaugen, Waschen mit n-Hexan und Trocknen im Vakuum erhielt man 173,5 g steingraues Produkt vom Fp. : 100 bis 101 °C.
Laut DC ist die Verbindung einheitlich.

b) N-Phenyl-benz[c, d]-indol-2-on

135,75 g (0,4 Mol) N-α-Naphthyl-N-phenyl-carbamidsäure-phenylester wurden in 317 g o-Dichlorbenzol gelöst ; die Lösung wurde zu 106,7 g (0,8 Mol) Aluminiumchlorid in 304 g o-Dichlorbenzol bei 160 °C in einer Stunde zugetropft. Man ließ 5 h bei 160 °C nachrühren, heizte dann auf 180 °C auf und ließ weitere 1,5 Stunden rühren. Nach Abkühlen trug man auf 400 g Eis aus, erhitzte die Mischung auf 65 °C, saugte zur Klärung über ein Papierfilter ab und trennte die Phasen. Die o-Dichlorbenzollösung wurde erneut bei 65 bis 70 °C mit 500 ml Wasser gewaschen, dann wurde nach Zusatz von 50 ml Toluol das Lösungsmittel im Vakuum abdestilliert.

Der Rückstand wurde im Hochvakuum destilliert. Bei 198 bis 205 °C/1,1 mbar gingen 75,3 g eines zähen gelb-roten Öls über, welches nach mehreren Tagen im Vorlagekolben erstarrte.

Das Produkt wurde aus Toluol/n-Hexan umkristallisiert. Man erhielt grünliche Nädelchen vom Fp. : 103,5 bis 105 °C.

Beispiel 17

a) N-(3-Methyl-phenyl)-N-α-naphthyl-carbaminsäure-phenylester

140.0 g (0.6 Mol) N-[3-Methyl-phenyl]-N-α-naphthylamin wurden in 470 g o-Dichlorbenzol vorgelegt und die Lösung unter Rühren auf 100 °C aufgeheizt. Dann tropfte man 98,28 g (0.63 Mol) Chlorameisensäurephenylester innerhalb einer Stunde zu. Nach beendeter Zugabe erhitzte man auf 120 °C, wobei eine Gasentwicklung auftrat. Das Reaktionsgemisch wurde 3,5 Stunden bei dieser Temperatur gerührt, danach war die Gasentwicklung beendet. Man engte die Lösung am Rotationsverdampfer bis zur Gewichtskonstanz ein, versetzte das zurückbleibende Öl mit Methanol und ließ über Nacht im Kühlschrank kristallisieren. Nach Absaugen, Waschen mit eiskaltem Methanol und Trocknen erhielt man 169.5 g braun-beige-farbige Kristalle vom Fp. 108-9 °C.

≙ 80 % der theoretischen Ausbeute

b) N-(3-Methyl-phenyl)-benz [c, d]-indol-2-on

70.68 g (0.2 Mol) N-(3-Methyl-phenyl)-N-α-naphthylcarbamidsäurephenylester wurden in 158 g o-Dichlorbenzol gelöst und die Lösung zu 66.67 g (0.5 Mol) Aluminiumchlorid in 190 g o-Dichlorbenzol bei 160 °C innerhalb einer Stunde zudosiert.

Man ließ 4 Stunden bei 160 °C nachrühren, kühlte auf Raumtemperatur ab und trug den Ansatz auf Eiswasser aus. Nach Klärfiltration wurden die Phasen getrennt, die wässrige Phase mit Methylenchlorid nachextrahiert, die vereinigten organischen Phasen zweimal mit Wasser und einmal mit 3 %iger Natriumhydrogencarbonatlösung gewaschen und über Natriumsulfat getrocknet. Nach Abdampfen des Lösungsmittels im Rotationsverdampfer wurde der Rückstand im Vakuum destilliert. Als Hauptfraktion erhielt man bei 195-204 °C/0,9 mbar ein gelbgrünes, fluorescierendes Öl, welches nach einigen Tagen in der Vorlage kristallin erstarrte. Man kristallisierte aus Toluol/n-Hexan um und erhielt grün-gelbe Nadeln vom Fp. 99-103 °C.

Beispiel 18 (zum Vergleich)

a) N-α-Naphthyl-carbamidsäure-phenylester

85.9 g (0.6 Mol) α-Naphthylamin wurden in 120 ml Toluol gelöst, die Lösung mit 38 ml Wasser und 56.0 g (0.63 Mol) 45 %iger Natronlauge versetzt und die Mischung unter Rühren auf 10 °C gekühlt. Man tropfte zu dem gut gerührten Gemisch innerhalb einer Stunde eine Lösung von 98.28 g (0.63 Mol) Chlorameisensäurephenylester in 78 ml Toluol. Durch externe Kühlung wurde die Temperatur zwischen 10 und 15 °C gehalten. Nach beendeter Zugabe wurde die entstandene Suspension noch 4h bei Raumtemperatur nachgerührt ; dann saugte man ab, wusch mit 1 Ltr. Wasser nach und trocknete im Vakuum. Ausbeute : 143.0 g ≙ 91 % der theoretischen Ausbeute. Lt. DC reines Produkt.

b) Benz[c, d]-indol-2-on

Zu einer Lösung von 133.34 g (1.0 Mol) Aluminiumchlorid in 625 ml o-Dichlorbenzol trug man bei 160 °C 105.3 g (0.4 Mol) N-α-Naphthyl-carbamidsäurephenylester innerhalb einer Stunde ein. Anschließend wurde 4h bei 160 °C nachgerührt. Dann kühlte man ab, hydrolysierte durch Austragen auf 500 g Eis und saugte das Zweiphasengemisch über ein Filter ab. Anschließend wurde die wässrige Phase abgetrennt, die organische Phase erneut mit 500 ml Wasser extrahiert, die vereinigten wäßrigen Phasen mit 200 ml o-Dichlorbenzol reextrahiert und die vereinigten organischen Phasen, nach Zugabe von 200 ml Toluol, bis zur Gewichtskonstanz im Rotationsverdampfer eingeengt. Der Rückstand, ein zähflüssiges Öl, wurde heiß in 600 ml 10 %iger Natronlauge gelöst, die Lösung filtriert und das Produkt durch Ansäuern mit Salzsäure bis pH 1-2 wieder ausgefällt. Nach erneutem Lösen in Natronlauge, Filtrieren und Ausfällen mit Salzsäure erhielt man nach Trocknen 38.1 g braunes Pulver. Laut HFC enthielt das Produkt 23.8 % Benz-[c, d]-indol-2-on.

Beispiel 19

N-[4-(benz-[c, d]-indol-2-on-1-yl)-phenyl]-benz-[c, d]-indol-2-on

10.99 g (82.4 mMol) $AlCl_3$ wurden in 60 ml o-Dichlorbenzol suspendiert und das Gemisch auf Rückflußtemperatur (178 °C) aufgeheizt, wobei Aluminiumchlorid in Lösung ging. Dann tropfte man innerhalb einer Stunde eine heiße Lösung von 4.95 g (8.24 mMol) p-Phenylen-diamin-(N,N'-di-α-naphthyl-N,N'-di-phenoxycarbonyl) in 60 ml o-Dichlorbenzol zu und ließ anschließend noch 5 Stunden unter Rückfluß kochen.

Die abgekühlte Lösung wurde auf 200 g Eis ausgetragen und das o-Dichlorbenzol mit Wasserdampf abdestilliert. Der Destillationssumpf wurde abgesaugt, mit Wasser gewaschen und im Trockenschrank getrocknet. Man erhielt 4.24 g Rohprodukt.

Nach Umkristallisation aus 400 ml DMF konnten 1.84 g dunkelbraune Kristalle vom Fp. > 365 °C ≙ 54 % der Theorie isoliert werden.

**Patentansprüche**

1. Verfahren zur Herstellung von N-substituierten Benz[c, d]-indol-2-onen (Naphtholactamen) der Formel

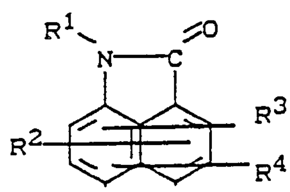

(VII)

worin

$R^1$ Phenyl, 2-, 3- oder 4-Pyridyl ; 2- oder 3-Pyrazinyl ; 2-, 4- oder 5-Pyrimidinyl ; 3- oder 4-Pyridazinyl ; 2-Triazinyl ; 2-, 3-, 4-, 5-, 6-, 7- oder 8-Chinolinyl ; 1-, 3-, 4-, 5-, 6-, 7- oder 8-Isochinolinyl ; 2-, 3-, 5-, 6-, 7- oder 8-Chinoxalinyl ; 2-, 4-, 5-, 6-, 7- oder 8-Chinazolinyl ; 1,8-Naphthyridin-2-, -3-, -4-, -5-, -6- oder -7-yl ; 3-, 4-, 5-, 6-, 7- oder 8-Cinnolinyl ; 1-, 4-, 5-, 6-, 7- oder 8- Phthalazinyl ; 2-, 4-, 6- oder 7-Pteridinyl ; 1-, 2-, 3-, 4-, 6-, 7-, 8- oder 9-Phenazinyl ; 1-, 2-, 3-, 4-, 5-, 6-, 7-, 8- oder 9-Acridinyl ; 1-, 2-, 3-, 4-, 6-, 7-, 8-, 9- oder 10-Phenanthridinyl ; 2- oder 3-Pyrrolyl ; 1-, 2- oder 4-Imidazolyl ; 1-, 2-, 3-, 4-, 5-, 6- oder 7-Indolyl ; 3-, 4-, 5-, 6- oder 7-Indazolyl ; 2-, 6- oder 8-Purinyl ; 2- oder 3-Thienyl ; 2- oder 3-Furyl ; 2-, 3-, 4-, 5-, 6- oder 7-Benzo-[b]-thienyl ; 1-, 2-, 3-, 4-, 6-, 7-, 8- oder 9-Thianthrenyl ; 2-, 3-, 4-, 5-, 6- oder 7-Benzo[b]-furanyl ; 1-, 2-, 3-, 4-, 5-, 6-, 7- oder 8-Xanthenyl ; 2-, 4- oder 5-Thiazolyl ; 2-, 4- oder 5-Oxazolyl ; 3-Furazanyl ; 1-, 2-, 3-, 4-, 6-, 7-, 8- oder 9-Phenoxazinyl ; 1-, 2-, 3-, 4-, 6-, 7-, 8- oder 9-Phenothiazinyl oder $-CH_2-R^8$ darstellt, worin $R^8$ Wasserstoff, geradkettiges oder verzweigtes $C_1$-$C_3$-Alkyl oder Phenyl bedeutet und

$R^2$, $R^3$ und $R^4$ unabhängig voneinander für Wasserstoff, geradkettiges oder verzweigtes $C_1$-$C_{20}$-Alkyl, $C_4$-$C_8$-Cycloalkyl, Phenyl, Naphthyl, Anthryl, Biphenyl, Benzyl, Phenyl-ethyl, Naphthyl-methyl, Naphthyl-ethyl, Anthryl-methyl, Anthryl-ethyl, Halogen, Cyano, Hydroxy, Formyl, $C_1$-$C_{10}$-Alkylcarbonylamino, Phenylamino, $C_1$-$C_{10}$-Alkoxycarbonyl, $C_1$-$C_{10}$-Alkoxycarbonyloxy, Phenyloxycarbonyl, Phenylcarbonyloxy, $C_1$-$C_{10}$-Alkylcarbonyl, Phenylcarbonyl, $C_1$-$C_{10}$-Alkoxy, Phenyloxy, $C_1$-$C_{10}$-Alkylthio, Benzylthio, Phenylthio, $C_1$-$C_{10}$-Alkoxycarbonyloxy, $C_1$-$C_{10}$-Acylamino, Di-$C_1$-$C_{10}$-alkylamino, $C_1$-$C_{10}$-Alkylphenylamino, $C_1$-$C_{10}$-Alkylaminocarbonyloxy, $C_1$-$C_{10}$-Alkylsulfonylamino, Ureido, N-$C_1$-$C_{10}$-Alkylureido, Phenyloxycarbonylamino, $C_1$-$C_{10}$-Alkoxycarbonylamino, Carbamoyl, N-$C_1$-$C_{10}$-Alkyl-carbamoyl, Di-N,N-$C_1$-$C_{10}$-alkylcarbamoyl, N-$C_1$-$C_{10}$-Alkyl-N-phenylcarbamoyl, Sulfamoyl, N-$C_1$-$C_{10}$-Alkyl-sulfamoyl, Di-N,N-$C_1$-$C_{10}$-alkyl-sulfamoyl, Phenyloxysulfonyl, $C_1$-$C_{10}$-Alkoxysulfonyl, Nitro, $C_1$-$C_{10}$-Alkylsulfonyl, Benzylsulfonyl oder Phenylsulfonyl stehen, wobei weiterhin zwei der Reste $R^2$, $R^3$ und $R^4$ gemeinsam einen anellierten aromatischen oder cycloaliphatischen Ring darstellen können,

dadurch gekennzeichnet, daß man N-subtituierte N-α-Naphthyl-carbamidsäureester der Formel

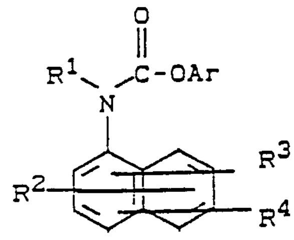

(I)

in der

$R^1$ bis $R^4$ die genannte Bedeutung haben und

Ar Phenyl, das einfach oder mehrfach durch $C_1$-$C_4$-Alkyl, $C_1$-$C_4$-Alkoxy, Fluor, Chlor, Brom oder Cyano substituiert sein kann, oder Naphthyl bedeutet, in Gegenwart von 1-5 Mol eines Friedel-Crafts-Katalysators pro Mol des Carbamidsäureesters in einem für Friedel-Crafts-Reaktionen geeigneten Lösungsmittel bei erhöhter Temperatur umsetzt.

2. Verfahren nach Anspruch 1, dadurch gekennzeichnet, daß Carbamidsäureester eingesetzt werden, die anstelle der Reste $R^2$, $R^3$ und $R^4$ die Reste $R^{12}$, $R^{13}$ und $R^{14}$ tragen, die unabhängig voneinander Wasserstoff, Fluor, Chlor, Brom, Cyano, Phenyloxy-carbonylamino, $C_1$-$C_4$-Alkyl oder $C_1$-$C_4$-Alkoxy bedeuten.

3. Verfahren nach Ansprüchen 1 und 2, dadurch gekennzeichnet, daß man als Friedel-Crafts-Katalysator Aluminiumchlorid oder Aluminiumbromid verwendet.

4. Verfahren nach Ansprüchen 1 bis 3, dadurch gekennzeichnet, daß man den Friedel-Crafts-Katalysator in einer Menge von 1,5-3,5 Mol pro Mol Carbamidsäureestergruppe einsetzt.

5. Verfahren nach Ansprüchen 1 bis 4, dadurch gekennzeichnet, daß man die Reaktion bei 100-250 °C durchführt.

6. Verfahren nach Ansprüchen 1 bis 5, dadurch gekennzeichnet, daß man den Carbamidsäureester als Schmelze zu einer Lösung des Friedel-Carfts-Katalysators zudosiert.

7. Verfahren zur Herstellung von N-substituierten Benz[c, d]-indol-2-onen (Naphtholactamen) entsprechend Anspruch 1, dadurch gekennzeichnet, daß man in einer ersten Stufe ein N-substituiertes α-Naphthylamin der Formel

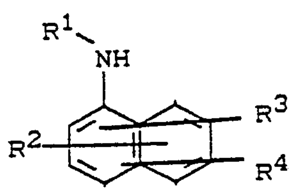

(V)

worin $R^1$ und $R^4$ die in Anspruch 1 genannte Bedeutung haben, mit 1,0-1,2 Mol eines Chlorameisensäureesters der Formel

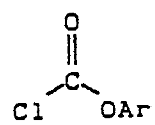

(IV)

worin Ar die in Anspruch 1 genannte Bedeutung hat, pro Mol N-substituierten α-Naphthylamins bei erhöhter Temperatur in einem für Friedel-Crafts-Reaktionen geeigneten Lösungsmittel ohne Hilfsbase umsetzt und diese Lösung, gegebenenfalls nach Aufkonzentrieren, in einer zweiten Stufe zu einer Lösung von Aluminiumhalogenid im gleichen Lösungsmittel bei erhöhter Temperatur zusetzt und dann weiter zum N-substituierten Benz[c, d]-indol-2-on (Naphtholactam) nach Anspruch 1 umsetzt.

**Claims**

1. Process for the preparation of N-substituted benz[c, d]-indol-2-ones (naphtholactams) of the formula

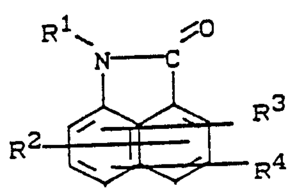

(VII)

in which

$R^1$ represents phenyl, 2-, 3- or 4-pyridyl ; 2- or 3-pyrazinyl ; 2-, 4- or 5-pyrimidinyl ; 3- or 4-pyridazinyl ; 2-triazinyl ; 2-, 3-, 4-, 5-, 6-, 7- or 8-quinolinyl ; 1-, 3-, 4-, 5-, 6-, 7- or 8-isoquinolinyl ; 2-, 3-, 5-, 6-, 7- or 8-quinoxalinyl ; 2-, 4-, 5-, 6-, 7- or 8-quinazolinyl ; 1,8-naphthyridin-2-, -3-, -4-, -5-, -6- or -7-yl ; 3-, 4-, 5-, 6-, 7- or 8-cinnolinyl ; 1-, 4-, 5-, 6-, 7- or 8-phthalazinyl ; 2-, 4-, 6- or 7-pteridinyl ; 1-, 2-, 3-, 4-, 6-, 7-, 8- or 9-phenazinyl ; 1-, 2-, 3-, 4-, 5-, 6-, 7-, 8- or 9-acridinyl ; 1-, 2-, 3-, 4-, 6-, 7-, 8-, 9- or 10-phenanthridinyl ; 2- or 3-pyrrolyl ; 1-, 2- or 4-imidazolyl ; 1-, 2-, 3-, 4-, 5-, 6- or 7-indolyl ; 3-, 4-, 5-, 6- or 7-indazolyl ; 2-, 6- or 8-purinyl ; 2- or 3-thienyl ; 2- or 3-furyl ; 2-, 3-, 4-, 5-, 6- or 7-benzo-[b]-thienyl ; 1-, 2-, 3-, 4-, 6-, 7-, 8- or 9-thian-threnyl ; 2-, 3-, 4-, 5-, 6- or 7-benzo-[b]-furanyl ; 1-, 2-, 3-, 4-, 5-, 6-, 7- or 8-xanthenyl ; 2-, 4- or 5-thiazolyl ; 2-, 4- or 5-oxazolyl ; 3-furazanyl ; 1-, 2-, 3-, 4-, 6-, 7-, 8- or 9-phenoxazinyl ; 1-, 2-, 3-, 4-, 6-, 7-, 8- or 9-phenothiazinyl or $—CH_2—R^8$ in which $R^8$ denotes hydrogen, straight-chain or branched $C_1$-$C_3$-alkyl or phenyl, and

$R^2$, $R^3$ and $R^4$, independently of one another, represent hydrogen, straight-chain or branched $C_1$-$C_{20}$-alkyl, $C_4$-$C_8$-cycloalkyl, phenyl, naphthyl, anthryl, biphenyl, benzyl, phenyl-ethyl, naphthyl-methyl, naphthyl-ethyl, anthryl-methyl, anthryl-ethyl, halogen, cyano, hydroxyl, formyl, $C_1$-$C_{10}$-alkylcarbonylamino, phenylamino, $C_1$-$C_{10}$-alkoxycarbonyl, $C_1$-$C_{10}$-alkoxycarbonyloxy, phenyloxycarbonyl, phenylcarbonyloxy, $C_1$-$C_{10}$-alkylcarbonyl, phenylcarbonyl, $C_1$-$C_{10}$-alkoxy, phenyloxy, $C_1$-$C_{10}$-alkylthio, benzylthio, phenylthio, $C_1$-$C_{10}$-alkoxycarbonyloxy, $C_1$-$C_{10}$-acylamino, di-$C_1$-$C_{10}$-alkylamino, $C_1$-$C_{10}$-alkylphenylamino, $C_1$-$C_{10}$-alkylaminocarbonyloxy, $C_1$-$C_{10}$-alkylsulphonylamino, ureido, N-$C_1$-$C_{10}$-alkylureido, phenyloxycarbonylamino, $C_1$-$C_{10}$-alkoxycarbonylamino, carbamoyl, N-$C_1$-$C_{10}$-alkyl-carbamoyl, di-N,N-$C_1$-$C_{10}$-alkylcarbamoyl, N-$C_1$-$C_{10}$-alkyl-N-phenylcarbamoyl, sulphamoyl, N-$C_1$-$C_{10}$-alkylsulphamoyl, di-N,N-$C_1$-$C_{10}$-alkyl-sulphamoyl, phenyloxysulphonyl, $C_1$-$C_{10}$-alkoxysulphonyl, nitro, $C_1$-$C_{10}$-alkylsulphonyl, benzylsulphonyl or phenylsulphonyl, it also being possible for two of the radicals $R^2$, $R^3$ and $R^4$ together to represent a fused aromatic or cycloaliphatic ring,
characterized in that N-substituted N-α-naphthyl-carbamates of the formula

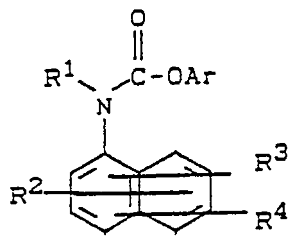
(I)

in which

$R^1$ to $R^4$ have the meaning mentioned and

Ar denotes phenyl, which can be monosubstituted or polysubstituted by $C_1$-$C_4$-alkyl, $C_1$-$C_4$-alkoxy, fluorine, chlorine, bromine or cyano, or denotes naphthyl,

are reacted in the presence of 1-5 mol of a Friedel-Crafts catalyst per mol of the carbamate, in a solvent is suitable- for Friedel-Crafts reactions, at elevated temperature.

2. Process according to Claim 1, characterized in that carbamates are used which carry the radicals $R^{12}$, $R^{13}$ and $R^{14}$ in place of the radicals $R^2$, $R^3$ and $R^4$, $R^{12}$, $R^{13}$ and $R^{14}$ independently of one another denoting hydrogen, fluorine, chlorine, bromine, cyano, phenyloxycarbonylamino, $C_1$-$C_4$-alkyl or $C_1$-$C_4$-alkoxy.

3. Process according to Claims 1 and 2, characterized in that aluminium chloride or aluminium bromide is used as the Friedel-Crafts catalyst.

4. Process according to Claims 1 to 3, characterized in that the Friedel-Crafts catalyst is used in a quantity of 1.5-3.5 mol per mol of carbamate group.

5. Process according to Claims 1 to 4, characterized in that the reaction is carried out at 100-250 °C.

6. Process according to Claims 1 to 5, characterized in that the carbamate is metered as a melt into a solution of the Friedel-Crafts catalyst.

7. Process according to Claim 1 for the preparation of N-substituted benz[c, d]indol-2-ones (naphtholactams), characterized in that, in a first step, an N-substituted α-naphthylamine of the formula

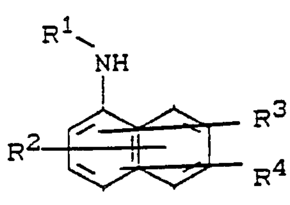
(V)

in which $R^1$ and $R^4$ have the meaning given in Claim 1, is reacted with 1.0-1.2 mol of a chloroformate of the formula

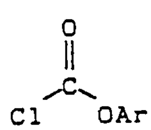
(IV)

in which Ar has the meaning given in Claim 1, per mol of N-substituted α-napththylamine, at elevated temperature, in a solvent suitable for Friedel-Crafts reactions, without an auxiliary base, and, in a second

step, this solution, if appropriate after concentration, is added to a solution of aluminium halide in the same solvent, at elevated temperature, and is then reacted further to give the N-substituted benz-[c, d]indol-2-one (naphtholactam) according to Claim 1.

**Revendications**

1. Procédé de production de benzo [c, d]-indole-2-ones (naphtolactames) N-substituées de formule

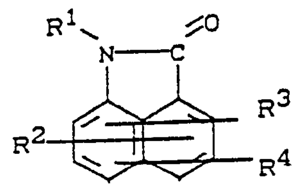

(VII)

dans laquelle

$R^1$ est un groupe phényle, 2-, 3- ou 4-pyridyle ; 2- ou 3-pyrazinyle ; 2-, 4- ou 5-pyrimidinyle ; 3- ou 4-pyridazinyle ; 2-triazinyle ; 2-, 3-, 4-, 5-, 6-, 7- ou 8-quinolinyle ; 1-, 3-, 4-, 5-, 6-, 7- ou 8-isoquinolinyle ; 2-, 3-, 5-, 6-, 7- ou 8-quinoxalinyle ; 2-, 4-, 5-, 6-, 7- ou 8-quinazolinyle ; 1,8-naphtyridine-2-, -3-, -4-, -5-, -6- ou -7-yle ; 3-, 4-, 5-, 6-, 7- ou 8-cinnolinyle ; 1-, 4-, 5-, 6-, 7- ou 8-phtalazinyle ; 2-, 4-, 6- ou 7-ptéridinyle ; 1-, 2-, 3-, 4-, 6-, 7-, 8- ou 9-phénazinyle ; 1-, 2-, 3-, 4-, 5-, 6-, 7-, 8- ou 9-acridinyle ; 1-, 2-, 3-, 4-, 6-, 7-, 8-, 9- ou 10-phénanthridinyle ; 2- ou 3-pyrrolyle ; 1-, 2- ou 4-imidazolyle ; 1-, 2-, 3-, 4-, 5-, 6- ou 7-indolyle ; 3-, 4-, 5-, 6- ou 7-indazolyle ; 2-, 6- ou 8-purinyle ; 2- ou 3-thiényle ; 2- ou 3-furyle ; 2-, 3-, 4-, 5-, 6- ou 7-benzo-[b]-thiényle ; 1-, 2-, 3-, 4-, 6-, 7-, 8- ou 9-thianthrényle ; 2-, 3-, 4-, 5-, 6- ou 7-benzo-[b]-furannyle ; 1-, 2-, 3-, 4-, 5-, 6-, 7- ou 8-xanthényle ; 2-, 4- ou 5-thiazolyle ; 2-, 4- ou 5-oxazolyle ; 3-furazanyle ; 1-, 2-, 3-, 4-, 6-, 7-, 8- ou 9-phénoxazinyle ; 1-, 2-, 3-, 4-, 6-, 7-, 8- ou 9-phénothiazinyle ou un groupe $—CH_2—R^8$ dans lequel $R^8$ est l'hydrogène, un radical alkyle en $C_1$ à $C_3$ à chaîne droite ou ramifiée ou un radical phényle et

$R^2$, $R^3$ et $R^4$ représentent, indépendamment les uns des autres, l'hydrogène, un groupe alkyle en $C_1$ à $C_{20}$ à chaîne droite ou à chaîne ramifiée, un groupe cycloalkyle en $C_4$ à $C_8$, un groupe phényle, naphtyle, anthryle, biphényle, benzyle, phényl-éthyle, naphtyl-méthyle, naphtyl-éthyle, anthryl-méthyle, anthryl-éthyle, un halogène, un groupe cyano, hydroxy, formyle, (alkyle en $C_1$ à $C_{10}$)-carbonylamino, phénylamino, (alkoxy en $C_1$ à $C_{10}$)-carbonyle, (alkoxy en $C_1$ à $C_{10}$) -carbonyloxy, phényloxycarbonyle, phénylcarbonyloxy, (alkyle en $C_1$ à $C_{10}$) -carbonyle, phénylcarbonyle, (alkoxy en $C_1$ à $C_{10}$), phényloxy, alkylthio en $C_1$ à $C_{10}$, benzylthio, phénylthio, (alkoxy en $C_1$ à $C_{10}$)-carbonyloxy, acylamino en $C_1$ à $C_{10}$, di(alkyle en $C_1$ à $C_{10}$)amino, (alkyle en $C_1$ à $C_{10}$)-phénylamino, (alkyle en $C_1$ à $C_{10}$)-aminocarbonyloxy, (alkyle en $C_1$ à $C_{10}$)-sulfonylamino, uréido, N-(alkyle en $C_1$ à $C_{10}$) uréido, phényloxycarbonylamino, (alkoxy en $C_1$ à $C_{10}$)-carbonylamino, carbamoyle, N(alkyle en $C_1$ à $C_{10}$)-carbamoyle, di-N,N-(alkyle en $C_1$ à $C_{10}$)-carbamoyle, N(alkyle en $C_1$ à $C_{10}$)-N-phénylcarbamoyle, sulfamoyle, N(alkyle en $C_1$ à $C_{10}$)-sulfamoyle, di-N,N(alkyle en $C_1$ à $C_{10}$)-sulfamoyle, phényloxysulfonyle, (alkoxy en $C_1$ à $C_{10}$)-sulfonyle, nitro, (alkyle en $C_1$ à $C_{10}$)-sulfonyle, benzylsulfonyle ou phénylsulfonyle, et en outre deux des restes $R^2$, $R^3$ et $R^4$ peuvent former conjointement un noyau aromatique ou cycloaliphatique condensé, qui est caractérisé en ce qu'on fait réagir des esters d'acides N-α-naphtyl-carbamiques substitués sur l'azote, de formule

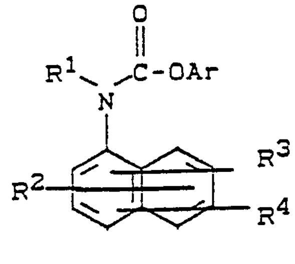

(I)

dans laquelle

$R^1$ à $R^4$ ont la définition mentionnée ci-dessus et

Ar est un groupe phényle, qui peut être substitué une ou plusieurs fois par des substituants alkyle en $C_1$ à $C_4$, alkoxy en $C_1$ à $C_4$, fluoro, chloro, bromo ou cyano, ou un groupe naphtyle,

en présence de 1 à 5 moles d'un catalyseur de Friedel-Crafts par mole de l'ester d'acide carbamique, à une température élevée dans un solvant qui convient pour des réactions de Friedel-Crafts.

2. Procédé suivant la revendication 1, caractérisé en ce qu'on utilise des esters d'acide carbamique qui portent à la place des restes $R^2$, $R^3$ et $R^4$ les restes $R^{12}$, $R^{13}$ et $R^{14}$ qui représentent, indépendamment les uns des autres, de l'hydrogène, du fluor, du chlore, du brome, un groupe cyano, phényloxycarbonylamino, alkyle en $C_1$ à $C_4$ ou alkoxy en $C_1$ à $C_4$ ;

3. Procédé suivant les revendications 1 et 2, caractérisé en ce qu'on utilise comme catalyseur de Friedel-Crafts du chlorure d'aluminium ou du bromure d'aluminium.

4. Procédé suivant les revendications 1 à 3, caractérisé en ce qu'on utilise le catalyseur de Friedel-Crafts en une quantité de 1,5-3,5 moles par mole de groupe ester d'acide carbamique.

5. Procédé suivant les revendications 1 à 4, caractérisé en ce qu'on conduit la réaction à 100-250 °C.

6. Procédé suivant les revendications 1 à 5, caractérisé en ce qu'on ajoute l'ester d'acide carbamique à l'état fondu à une solution du catalyseur de Friedel-Crafts.

7. Procédé de production de benzo [c, d]-indole-2-ones (naphtolactames) substituées sur l'azote suivant la revendication 1, caractérisé en ce qu'on fait réagir dans une première étape une α-naphtylamine substituée sur l'azote de formule

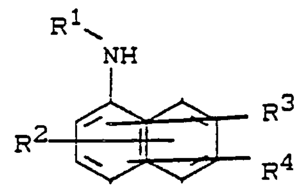

(V)

dans laquelle $R^1$ et $R^4$ ont la définition mentionnée dans la revendication 1, avec 1,0-1,2 mole d'un ester d'acide chloroformique de formule

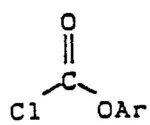

(IV)

dans laquelle Ar a la définition mentionnée dans la revendication 1, par mole d'α-naphtylamine substituée sur l'azote à température élevée dans un solvant convenable pour des réactions de Friedel-Crafts sans base auxiliaire et on ajoute cette solution, éventuellement après concentration, dans une seconde étape à une solution d'halogénure d'aluminium dans le même solvant à température élevée, puis on poursuit la réaction jusqu'à l'obtention de la benzo [c, d] -indole-2-one (naphtolactame) substituée sur l'azote suivant la revendication 1.